Europäisches Patentamt

European Patent Office

Office européen des brevets

(19)

(11) Veröffentlichungsnummer: **0 188 696**
**B1**

(12)
# EUROPÄISCHE PATENTSCHRIFT

(45) Veröffentlichungstag der Patentschrift: **04.04.90**

(21) Anmeldenummer: **85114779.3**

(22) Anmeldetag: **21.11.85**

(51) Int. Cl.⁵: **A 61 M 1/00**

(54) **Druckanzeigender Behälterverschluss.**

(30) Priorität: **19.01.85 DE 3501664**

(43) Veröffentlichungstag der Anmeldung:
**30.07.86 Patentblatt 86/31**

(45) Bekanntmachung des Hinweises auf die
Patenterteilung:
**04.04.90 Patentblatt 90/14**

(84) Benannte Vertragsstaaten:
**AT BE CH DE FR GB IT LI LU NL SE**

(56) Entgegenhaltungen:
**EP-A-0 066 699**
**EP-A-0 068 824**
**FR-A-2 288 530**

(73) Patentinhaber: **Oriplast Gebr. Krayer GmbH,
Herstellung und Vertrieb medizinischer und
pharmazeutischer Artikel
Hermannstrasse (Industriegebiet)
D-6680 Neunkirchen (DE)**

(72) Erfinder: **Krayer, Rolf
Bahnhofstrasse 94
D-6601 Kleinblittersdorf 2 (DE)**
Erfinder: **Krayer, Paul
Holbeinstrasse 3
D-6606 Gersweiler (DE)**

(74) Vertreter: **Schönherr, Wolfgang et al
Patentanwälte Wolfgang Schönherr Dipl.-Ing.
Karl-Heinz Serwe Hawstrasse 28
D-5500 Trier (DE)**

Courier Press, Leamington Spa, England.

**Beschreibung**

Die Erfindung bezieht sich auf einen druckanzeigenden Behälterverschluß mit einer Membran, an deren Außenseite ein Anzeigestab exzentrisch angeordnet ist.

Ein derartiger Verschluß ist beispielsweise aus der EP—A—0068824 bekannt.

Ein derartiger Behälterverschluß findet Verwendung bei einer Vakuum-Saugflasche für medizinische Zwecke, insbesondere zum Absaugen von Blut und Sekreten.

Zur Verwendung des Behälterverschlusses wird der Behälterverschluß auf den zugehörigen Behälter aufgesetzt und der Behälter, in der Regel eine Glasflasche, evakuiert. Durch den Unterdruck in der Glasflasche wird die Membran des Behälterverschlusses angesaugt, so daß der auf der Membran angeordnete Anzeigestab um einen Winkel von beispielsweise fast 90° zur Seite schwenkt.

Somit kann auf einfache Weise festgestellt werden, ob ein Unterdruck in der Flasche vorhanden ist.

Dieser bekannte druckanzeigende Behälterverschluß läßt somit erkennen, ob in der Flasche ein Vakuum vorhanden ist, oder ob kein Vakuum vorhanden ist. Eine Abschätzung über den Betrag der Evakuierung der Flasche läßt dieser druckanzeigende Behälterverschluß nur schwer zu, da bereits bei einem sehr kleinen Unterdruck der Anzeigestab über den vollen Schwenkbereich geschwenkt wird.

Nachteilig ist weiterhin, daß, wie die Erfahrung gezeigt hat, der Anzeigestab des bekannten druckanzeigenden Behälterverschlusses nicht mehr in seine Ruhestellung zurückkehrt, wenn der druckanzeigende Behälterverschluß längere Zeit auf einer beispielsweise mit 755 mm/Hg. vorevakuierten Flasche angeordnet war. Es hat sich gezeigt, daß dann die Membran mit den zugeordneten Wandungen des Behälterverschlusses verklebt.

Bekannt ist weiterhin ein Differenzdruckanzeiger, der einen Ballon oder einen Schlauch in einem zylindrischen Gehäuse aufweist. Ist der Druck im Gehäuse niedriger als im Ballon, so bläht sich der Ballon auf und legt sich an der Innenwand des Gehäuses an. Die Höhe des Anlagerandes gibt mit Hilfe einer Skala am Gehäuse die Druckdifferenz an. Nachteilig ist bei diesem Differenzdruckanzeiger die zur Erzielung einer ausreichenden Genauigkeit notwendige große Länge des Gehäuses. Nachteilig ist schließlich, daß zur Erzielung eines gleichmäßigen Anlagerandes der Ballon oder der Schlauch unter hohem Aufwand hergestellt werden muß.

In ähnlicher Weise ist es bekannt, zur Anzeige des Unterdruckes in einer Baby-Nahrungsflasche einen Ballon anzuordnen, der mit der Außenluft in Verbindung steht. Tritt ein Unterdruck in der Baby-Flasche auf, so dehnt sich der Ballon aus. Mit Hilfe einer Skala an der Baby-Flasche kann anhand der Ausdehnung des Ballons die Größe des Unterdrucks abgelesen werden. Auch diese Vorrichtung ist sehr ungenau.

Bekannt ist weiterhin ein Druckanzeiger mit einer Membran, der eine mechanisch wirkende Anzeigevorrichtung zugeordnet ist. Die Auslenkung der Membran bei einem Druckunterschied auf den beiden Seiten der Membran wird bei dieser Vorrichtung in eine Drehbewegung eines Zeigers umgewandelt. Der Druck kann so auf einer Skala abgelesen werden. Diese Vorrichtung ist sehr kompliziert aufgebaut und zur Erzielung einer annähernd genauen Anzeige nur mit großem Kostenaufwand herstellbar.

Es ist weiterhin eine Vorrichtung mit einer elastischen Membran zur Druckanzeige bekannt. Bei dieser Vorrichtung wird die Verschiebung der Membran über eine komplizierte optische Vorrichtung angezeigt. Diese Vorrichtung ist insgesamt nur sehr kostenaufwendig herstellbar.

Die Aufgabe der Erfindung besteht daher darin, einen druckanzeigenden Behälterverschluß der eingangs genannten Art derart weiterzubilden, daß die Schwenkbewegung des Anzeigestabes in etwa proportional zu der Größe des Unterdruckes verläuft und daß der Anzeigestab auch nach längerer Belastung der Membran wieder in seine Ruhestellung zurückkehrt.

Diese Aufgabe wird dadurch gelöst, daß der größte Durchmesser der etwa kreis- oder ellipsenförmigen, aus einem etwa rechtwinklig zur Längsrichtung des Stabes verlaufenden ersten Membranabschnitt und einem dazu in etwa einem Winkel von 45° verlaufenden zweiten, nach unten abgewinkelten, schrägen Membranabschnitt bestehenden Membran etwa doppelt so groß wie der Durchmesser des Anzeigestabes ist, daß der Anzeigestab derart auf dem ersten Membranabschnitt etwa auf dem größten Durchmesser angeordnet ist, daß der Anzeigestab einerseits am Rand des schrägen Membranabschnittes und andererseits in einem kleinen Abstand in dem dem schrägen Membranabschnitt abgewandten Rand des ersten Abschnittes steht, und daß in Richtung des größten Durchmessers gesehen die Länge des ersten Membranabschnittes etwas größer als die Länge des schrägen Membranabschnittes ist.

Vorteilhaft ist die Länge des ersten Membranabschnittes etwa um den Betrag des Abstandes des Anzeigestabes vom Rand des ersten Membranabschnittes größer als die Länge des schrägen Membranabschnittes. Vorzugsweise beträgt der Abstand des Anzeigestabes vom Rand des ersten Membranabschnittes etwa ein Viertel des Durchmessers des Anzeigestabes.

Bei einer vorteilhaften Ausführungsform ist die Membran am Ende einer einseitig offenen Ausnehmung des Behälterverschlusses angeordnet, wobei die sich an den Rand der Membraninnenseite anschließenden Wandabschnitte des Behälterverschlusses im wesentlichen parallel zur Längsrichtung des Anzeigestabes verlaufen. Vorzugsweise ist die Außenseite des schrägen Membranabschnittes geradlinig bis zum Rand des Behälterverschlusses fortgesetzt ausgebildet.

Die Wandstärke des sich an den schrägen Membranabschnitt anschliessenden Randabschnittes des Behälterverschlusses in Richtung des größten Durchmessers ist vorzugsweise etwa gleich dem Durchmesser des Anzeigestabes.

Bei einer vorteilhaften Ausführungsform sind zwei einander diametral gegenüberliegende Membranen mit exzentrischen Anzeigestäben im Behälterverschluß angeordnet. Vorzugsweise weisen der bzw. die Anzeigestäbe etwa kreisförmigen, elliptischen oder quadratischen Querschnitt auf. Vorzugsweise besteht der Behälterverschluß einstückig mit den Membranen und den Anzeigestäben aus Gummi.

Bei einer weiteren vorteilhaften Ausführungsform ist den Anzeigestäben eine eine Skala tragende, die Anzeigestäbe überragende Schutzplatte zugeordnet. Vorzugsweise sind zwei im Abstand zueinander stehende Schutzplatten beiderseits des bzw. der Anzeigestäbe vorgesehen.

Die Erfindung ist in den Zeichnungen beispielhaft dargestellt. Es zeigen:

Fig. 1 einen erfindungsgemäßen druckanzeigenden Behälterverschluß im Schnitt,

Fig. 2 eine andere Ausführungsform eines druckanzeigenden Behälterverschlusses im Schnitt,

Fig. 3 den Behälterverschluß nach Fig. 1 in Unteransicht,

Fig. 4 den Behälterverschluß nach Fig. 2 in Unteransicht,

Fig. 5 den Behälterverschluß nach Fig. 2 in Seitenansicht in Ruhestellung und

Fig. 6 den Behälterverschluß nach Fig. 2 in Seitenansicht mit nach außen geschwenkten Anzeigestäben in Folge eines auf den Behälterverschluß wirkenden Unterdruckes.

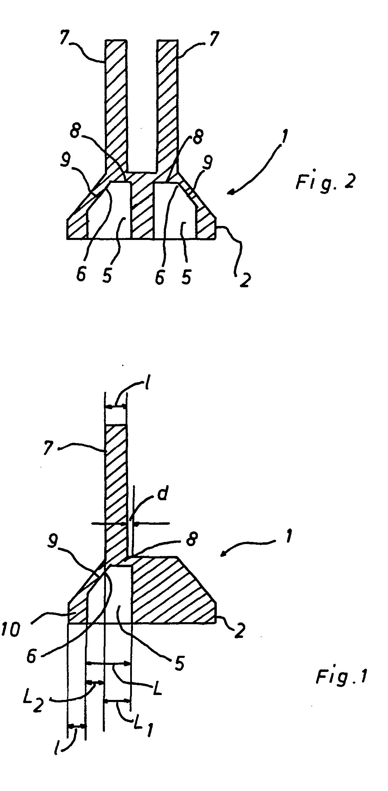

Nach den Fig. 1 bis 6 ist ein druckanzeigender Behälterverschluß 1 kegelstumpfförmig ausgebildet.

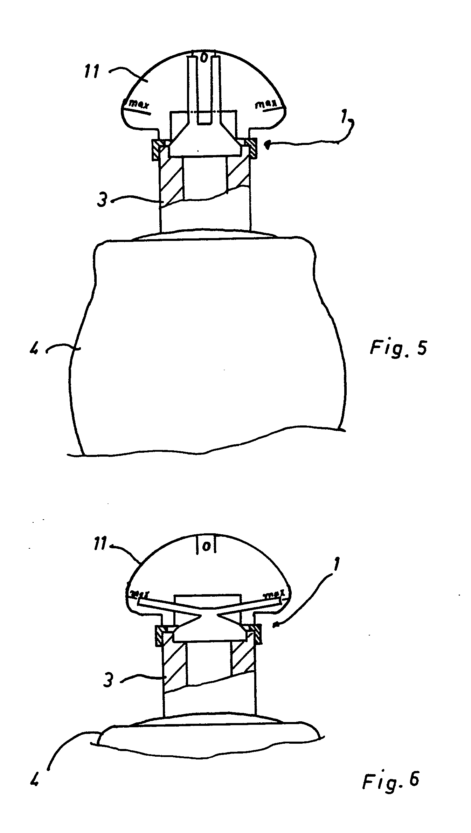

Der Behälterverschluß 1 hat einen unteren, zylinderförmigen Abschnitt 2, der zur Befestigung in einem Anschlußteil 3 eines zu evakuierenden Behälters, beispielsweise einer Saugflasche 4, dient, wie dies in den Fig. 5 und 6 gezeigt ist.

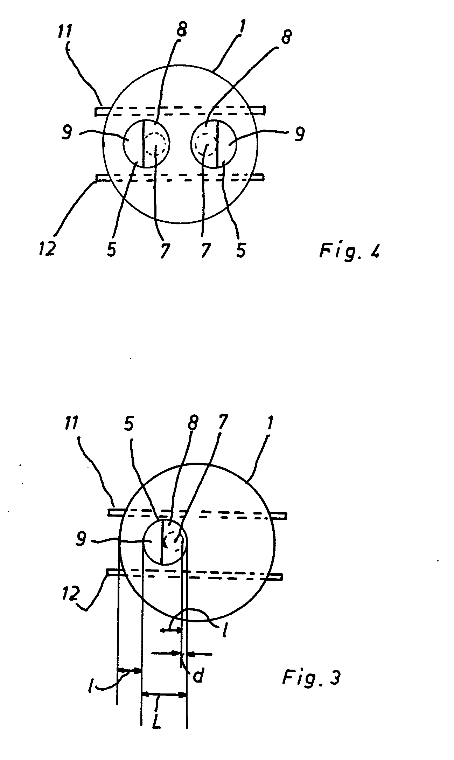

Nach den Fig. 1 und 3 weist der Behälterverschluß an seiner Unterseite eine sacklochartige Ausnehmung 5 auf, die an der Oberseite des Behälterverschlusses 1 durch eine Membran 6 verschlossen ist. Die Membran 6 trägt an ihrer Außenseite einen exzentrisch angeordneten Anzeigestab 7.

Die Ausführungsform eines Behälterverschlusses nach den Fig. 2, 4, 5 und 6 unterscheidet sich von der Ausführungsform nach den Fig. 1 und 3 nur dadurch, daß an Stelle einer Ausnehmung 5 mit einer Membran 6 und einem Anzeigestab 7 jeweils zwei einander diametral gegenüberliegende Membranen 5 mit exzentrischen Anzeigestäben 6 vorgesehen sind. Die Wirkungsweise der Membranen mit Anzeigestäben ist jeweils gleich.

Wie die Fig. 1 und 2 weiter erkennen lassen, hat die Membran 6 einen ersten, etwa rechtwinklig zur Längsrichtung des Anzeigestabes 7 verlaufenden Membranabschnitt 8 und einen dazu in etwa einem Winkel von 45° verlaufenden, zweiten, nach unten abgewinkelten, schrägen Membranabschnitt 9.

Wie die Fig. 1 bis 4 zeigen, ist der Anzeigestab 7 jeweils auf dem ersten Membranabschnitt 8 derart angeordnet, daß er einerseits am Rand des schrägen Membranabschnittes 9 und andererseits in einem kleinen Abstand d zum Rand des ersten Membranabschnittes 8 steht.

Der größte Durchmesser L der Membran 6 bzw. der Durchmesser der Ausnehmung 5 ist etwa doppelt so groß wie der Durchmesser 1 des Anzeigestabes. Weiterhin ist die Länge $L_1$ des Membranabschnittes 8 etwa um den Betrag des Abstandes d des Anzeigestabes 7 vom Rand des ersten Membranabschnittes 8 größer als die Länge $L_2$ des schrägen Membranabschnittes 9. Der Abstand d des Anzeigestabes 7 vom Rand des ersten Membranabschnittes 8 beträgt etwa ein Viertel des Durchmessers 1 des Anzeigestabes.

Wie die Figuren weiter erkennen lassen, ist die Außenseite des schrägen Membranabschnittes 9 geradlinig bis zum Rand bzw. bis zum zylinderförmigen Arbschnitt 2 des Behälterverschlusses 1 fortgesetzt ausgebildet, wobei der sich daran anschließende Randabschnitt 10 eine Wandstärke aufweist, die etwa gleich dem Durchmesser 1 des Anzeigestabes 7 ist. Durch die geradlinige Fortsetzung der Außenseite des schrägen Membranabschnittes 9 geht dieser Membranabschnitt stetig in den dickeren Randabschnitt 10 über.

In Ruhestellung, d. h. bei gleichem Druck auf beiden Seiten der Membran 6, stehen die Anzeigestäbe 7 in Richtung der Verlängerung der Ausnehmungen 5 geradlinig nach oben, wie dies die Fig. 1, 2 und 5 zeigen. Wirkt jedoch auf die Innenseite der Membran ein Unterdruck, so wird die Membran 6 nach innen in die Ausnehmung 5 gezogen, wodurch der Anzeigestab 7 in Abhängigkeit vom Unterdruck zur Seite schwenkt, wie dies in Fig. 6 gezeigt ist.

Durch die oben beschriebene Ausbildung der beiden Membranabschnitte 8 und 9 der Membran 6 wird dabei gewährleistet, daß das Maß der Schwenkung des Anzeigestabes 7 etwa proportional dem an der Innenseite der Membran 6 wirkenden Unterdruck ist. Außerdem wird gewährleistet, daß der Anzeigestab 7 nicht so weit zur Seite schwenken kann, daß die Innenseite des zugeordneten Membranabschnittes 8 mit der zugeordneten Wandung der Ausnehmung 5 des Behälterverschlusses 1 verklebt, so daß auch bei längerer Standzeit bei Fortfall des Unterdruckes der Anzeigestab 7 in seine Ruhestellung zurückkehrt.

Wie die Fig. 3 bis 6 zeigen, sind beiderseits der Anzeigestäbe 7 im Abstand und parallel zueinander verlaufende Schutzplatten 11 und 12 mit dem Behälterverschluß 1 bzw. mit dem Anschlußteil 3 der Saugflasche 4 verbunden. Die Schutzplatten 11 und 12 überragen die Anzeigestäbe 7, so daß eine Beschädigung der Anzeigestäbe vermieden wird. Eine der Schutzplatten 11 und 12, die aus einem durchsichtigen Werkstoff bestehen, trägt eine Skala, so daß der in der Saugflasche 4 vorhandene Unterdruck an Hand des als Zeiger wirkenden Anzeigestabes 7 auf der Skala abgelesen werden kann.

**Patentansprüche**

1. Druckanzeigender Behälterverschluß (1) mit einer Membran (6), an deren Außenseite ein Anzeigestab (7) exzentrisch angeordnet ist, dadurch gekennzeichnet, daß der größte Durchmesser (L) der etwa kreis- oder ellipsenförmigen, au einem etwa rechtwinklig zur Längsrichtung des Anzeigestabes (7) verlaufenden Membranabschnitt (8) und einem dazu in einem Winkel von etwa 45° verlaufenden, zweiten, nach unten abgewinkelten, schrägen Membranabschnitt (9) bestehenden Membran (6) etwa doppelt so groß wie der Durchmesser (1) des Anzeigestabes ist, daß der Anzeigestab derart auf dem ersten Membranabschnitt etwa auf dem größten Durchmesser angeordnet ist, daß der Anzeigestab einerseits am Rand des schrägen Membranabschnittes und andererseits in einem kleinen Abstand (d) zu dem dem schrägen Membranabschnitt abgewandten Rand des ersten Membranabschnittes steht, und daß in Richtung des größten Durchmessers gesehen die Länge (L₁) des ersten Membranabschnittes größer als die Länge (L₂) des schrägen Membranabschnittes ist.

2. Behälterverschluß nach Anspruch 1, dadurch gekennzeichnet, daß die Länge (L₁) des ersten Membranabschnittes (8) etwa um den Betrag des Abstandes (d) des Anzeigestabes (7) von dem dem schrägen Membranabschnitt abgewandten Rand des ersten Membranabschnittes größer als die Länge (L₂) des schrägen Membranabschnittes (9) ist.

3. Behälterverschluß nach einem der Ansprüche 1 oder 2, dadurch gekennzeichnet, daß der Abstand (d) des Anzeigestabes (7) von dem dem schrägen Membranabschnitt abgewandten Rand des ersten Membranabschnittes (8) etwa ein Viertel des Durchmessers (1) des Anzeigestabes beträgt.

4. Behälterverschluß nach einem der Ansprüche 1 bis 3, dadurch gekennzeichnet, daß die Membran (6) am Ende einer einseitig offenen Ausnehmung (5) des Behälterverschlusses (1) angeordnet ist, wobei die sich an den Rand der Membraninnenseite anschließenden Wandabschnitte des Behälterverschlusses im wesentlichen parallel zur Längsrichtung des Anzeigestabes (7) verlaufen.

5. Behälterverschluß nach einem der Ansprüche 1 bis 4, dadurch gekennzeichnet, daß die Außenseite des schrägen Membranabschnittes (9) geradlinig bis zum Rand (2) des Behälterverschlusses (1) fortgesetzt ausgebildet ist.

6. Behälterverschluß nach einem der Ansprüche 1 bis 5, dadurch gekennzeichnet, daß die Wandstärke des sich an den schrägen Membranabschnitt (9) anschließenden Randabschnittes (10) des Behälterverschlusses (1) in Richtung des größten Durchmessers etwa gleich dem Durchmesser (1) des Anzeigestabes (7) ist.

7. Behälterverschluß nach einem der Ansprüche 1 bis 6, dadurch gekennzeichnet, daß zwei einander diametral gegenüberliegende Membranen (6) mit exzentrischen Anzeigestäben im Behälterverschluß (1) angeordnet sind.

8. Behälterverschluß nach einem der Ansprüche 1 bis 7, dadurch gekennzeichnet, daß der bzw. die Anzeigestäbe (7) etwa kreisförmigen, elliptischen oder quadratischen Querschnitt aufweisen und daß der Behälterverschluß (1) einstückig mit den Membranen (6) und den Anzeigestäben (7) aus Gummi besteht.

9. Behälterverschluß nach einem der Ansprüche 1 bis 8, dadurch gekennzeichnet, daß dem bzw. den Anzeigestäben (7) eine eine Skala tragende, die Anzeigestäbe überragende Schutzplatte (11) zugeordnet ist.

10. Behälterverschluß nach Anspruch 9, dadurch gekennzeichnet, daß zwei im Abstand zueinander stehende Schutzplatten (11, 12) beiderseits des bzw. der Anzeigestäbe (7) vorgesehen sind.

**Revendications**

1. Fermeture (1) de récipient avec indication de pression comportant une membrane (6) sur la face extérieure de laquelle une baguette indicatrice (7) est montée en position excentrique, caractérisée par le fait que le grand diamètre (L) de la membrane (6), composée d'une première portion (8), approximativement circulaire ou elliptique placée perpendiculairement à l'axe longitudinal de la barrette indicatrice (7) et d'une deuxième portion (9) oblique orientée vers le bas, faisant avec la première portion un angle d'environ 45°, est approximativement deux fois plus grand que le diamètre (1) de la baguette indicatrice par le fait que celle-ci est disposée de telle sorte sur la première portion de membrane, approximativement sur le grand diamètre, que la baguette indicatrice se trouve d'une part au bord de la portion oblique de la membrane et d'autre part, à faible distance du bord opposé à la portion oblique de la membrane, et par le fait que lorsqu'on regarde dans le sens du grand diamètre, la longueur (L₁) de la première portion de membrane est plus grande que la longueur (L₂) de la portion oblique de la membrane.

2. Fermeture de récipient selon la revendication 1, caractérisée par le fait que la longueur (L₁) de la première portion de membrane (8) est plus grande que la longueur (L₂) de la portion oblique de la membrane (9) d'une valeur correspondant à la distance (d) de la baguette indicatrice (7) au bord de la première portion de membrane, opposé à la portion oblique de la membrane.

3. Fermeture de récipient selon l'une quelconque des revendications 1 ou 2, caractérisée par le fait que la distance (d) de la baguette indicatrice (7) au bord de la première portion de membrane (8), opposé à la portion oblique de membrane, est d'environ le quart du diamètre (1) de la baguette indicatrice.

4. Fermeture de récipient selon l'une quelconque des revendications 1 à 3, caractérisée par le fait que la membrane (6) est disposée à l'extrémité d'une cavité (5) ouverte sur un côté de la fermeture de récipient (1), et la paroi de la fermeture de récipient qui se raccorde au bord de la face intérieure de la membrane est substantiellement parallèle à l'axe longitudinal de la baguette indicatrice (7).

5. Fermeture de récipient selon l'une quelconque des revendications 1 à 4, caractérisée par le fait que la face extérieure de la portion oblique de la membrane (9) se continue de façon rectiligne jusqu'au bord (2) de la fermeture du récipient (1).

6. Fermeture de récipient selon l'une quelconque des revendications 1 à 5, caractérisée par le fait que l'épaisseur de la paroi de la partie du bord (10) se raccordant à la portion oblique de membrane (9) de la fermeture de récipient (1) dans le sens du grand diamètre est approximativement égale au diamètre (1) de la baguette indicatrice (7).

7. Fermeture de récipient selon l'une quelconque des revendications 1 à 6, caractérisée par le fait que deux membranes (6) diamétralement opposées entre elles sont disposées dans la fermeture de récipient (1) munies de baguettes indicatrices en position excentrique.

8. Fermeture de récipient selon l'une quelconque des revendications 1 à 7, caractérisée par le fait que la ou les baguettes indicatrices (7) présentent une section approximativement circulaire, elliptique ou carrée et que la fermeture de récipient (1) est en une seule pièce en caoutchouc avec les membranes (6) et les baguettes indicatrices (7).

9. Fermeture de récipient selon l'une quelconque des revendications 1 à 8, caractérisée par le fait que la ou les baguettes indicatrices (7) sont surmontées d'une plaque de protection (11) portant une échelle.

10. Fermeture de récipient selon la revendication 9, caractérisée par le fait que deux plaques de protection (11, 12) distantes l'une de l'autre sont prévues de part et d'autre de la ou des baguettes indicatrices (7).

**Claims**

1. Container-closing means with pressure indication (1), including a membrane (6) with an indicator rod (7) eccentrically disposed at its outside, characterised in that the largest diameter (L) of the approximately circular or elliptical membrane (6), comprising a membrane section (8) positioned approximately rectangular to the longitudinal direction of the indicator rod (7) and, at an angle of approximately 45° thereto, of a second membrane section (9) which is angled downward and slanting, is approximately double the size of the diameter (1) of the indicator rod; that the indicator rod is displaced on the first membrane section approximately on the largest diameter in such a manner that the indicator rod is located on the one hand at the edge of the slanting membrane section and on the other hand at a small distance (d) from the edge of the first membrane section which edge is facing away from the slanting membrane section; and that as seen in the direction of the largest diameter the length ($L_1$) of the first membrane section is larger than the length ($L_2$) of the slanting membrane section.

2. Container-closing means according to claim 1, characterised in that the length ($L_1$) of the first membrane section (8) is larger by about the amount of the distance (d) of the indicator rod (7) from the edge facing away from the slanting membrane section of the first membrane section than the length ($L_2$) of the slanting membrane section (9).

3. Container-closing means according to one of the claims 1 or 2, characterised in that the distance (d) of the indicator rod (7) from the edge facing away from the slanting membrane section of the first membrane section (8) equals approximately one quarter of the diameter (1) of the indicator rod.

4. Container-closing means according to one of the claims 1 to 3, characterised in that the membrane (6) is disposed at the end of a singlesidedly open space (5) of the container-closing means (1) whereby the wall sections adjacent to the edge of the membrane inner side of the container-closing means are essentially parallel to the longitudinal direction of the indicator rod (7).

5. Container-closing means according to one of the claims 1 to 4, characterised in that the outside of the slanting membrane section (9) is formed to be in a straight line up to the edge (2) of the container-closing means (1).

6. Container-closing means according to one of the claims 1 to 5, characterised in that the wall thickness of the edge section (10) adjacent to the slanting membrane section (9) of the container-closing means (1) in the direction of the largest diameter approximately equals the diameter (1) of the indicator rod (7).

7. Container-closing means according to one of the claims 1 to 6, characterised in that two membranes (6) which are disposed diametrically opposite each other and include eccentric indicator rods, are disposed in the container-closing means.

8. Container-closing means according to one of the claims 1 to 7, characterised in that the indicator rod(s) (7) has/have an approximately circular, elliptical or square cross-section, and that the container-closing means (1) are made of rubber in one piece with the membranes (6) and the indicator rods (7).

9. Container-closing means according to one of the claims 1 to 8, characterised in that the indicator rod(s) (7) has/have a cover plate (11) to protrude the indicator rods and accommodating a scale.

10. Container-closing means according to claim 9, characterised in that two cover plates (11, 12) disposed at a distance from each other are provided on both sides of the indicator rod(s) (7).

Fig. 2

Fig. 1

Fig. 4

Fig. 3

Fig. 5

Fig. 6